# EUROPÄISCHE PATENTSCHRIFT

(11) **EP 0 883 439 B1**
(45) Veröffentlichungstag und Bekanntmachung des Hinweises auf die Patenterteilung: **20.06.2001**
(21) Anmeldenummer: 97903335.4
(22) Anmeldetag: 20.02.1997
(51) Int. Cl.: B01J 35/04, B01J 29/04, B01J 23/22, B01J 21/06

(54) **NETZKATALYSATOR AUF BASIS VON TITAN- ODER VANADIUMZEOLITHEN UND INERTEN NETZGEWEBEN ZUR BESCHLEUNIGUNG VON OXIDATIONSREAKTIONEN**
GRID CATALYST BASED ON TITANIUM ZEOLITE OR VANADIUM ZEOLITE AND ON INERT RETICULATED FABRICS FOR USE IN THE CATALYSIS OF OXIDATION REACTIONS
CATALYSEUR RETICULE A BASE DE ZEOLITES AU TITANE OU AU VANADIUM ET DE TISSUS RETICULES INERTES POUR L'ACCELERATION DE REACTIONS D'OXYDATION

(30) Priorität: 29.02.1996 DE 19607577
(43) Veröffentlichungstag der Anmeldung: 16.12.1998
(73) Patentinhaber: BASF AKTIENGESELLSCHAFT, 67056 Ludwigshafen (DE)
(72) Erfinder: MÜLLER, Ulrich, D-67434 Neustadt (DE); BRÖCKER, Franz, Josef, D-67061 Ludwigshafen (DE); GROSCH, Georg, Heinrich, D-67098 Bad Dürkheim (DE); PÜTTER, Hermann, D-67433 Neustadt (DE); SCHULZ, Michael, D-67067 Ludwigshafen (DE); RIEBER, Norbert, D-68259 Mannheim (DE); HARDER, Wolfgang, D-69469 Weinheim (DE)
(86) Internationale Anmeldenummer: EP9700804
(87) Internationale Veröffentlichungsnummer: WO9731711

(56) Entgegenhaltungen:
- EP-A- 0 388 094
- DD-A- 271 101
- Chem.-Ing.-Tech. 67, Band, Nr. 6, 1995, (Weinheim), Hans Peter Calis et al, "Anwendung von Zeolithen bei der selektiven katalytischen Reduktion von NOx in Industrieabgasen"
- Microporous Materials, Band, Nr. 5, 1995, Satoshi Yamazaki et al, "Synthesis of a mordenite mambrane on a stainless-steel filter and polytetrafluoroethylene plate substrates"

## Beschreibung

Die vorliegende Erfindung betrifft einen Netzkatalysator auf Basis von bestimmten Titanzeolithen und inerten Netzgeweben und seine Verwendung zur Beschleunigung von Oxidationsreaktionen. Weiterhin sind Gegenstand der vorliegenden Erfindung entsprechende Verfahren zur Herstellung von Epoxiden, von Wasserstoffperoxid und von Hydroxylaminen.

Von Titanzeolithen ist bekannt, daß sie sich als Katalysatoren für Oxidationsreaktionen eignen. So wird beispielsweise in der EP-A 100 119 (1) ein Verfahren beschrieben, womit Propen in Gegenwart von Lösungsmitteln mit wäßrigem Wasserstoffperoxid an Titanzeolithen zu Propylenoxid epoxidiert werden kann. Die Herstellung von Cyclohexanonoxim aus Cyclohexanon durch Umsetzung mit Ammoniak und Wasserstoffperoxid an Titansilikaliten lehrt die EP-A 208 311 (2) und die Hydroxylierung von Aromaten mittels Wasserstoffperoxid an synthetischen Zeolithen ist aus der GB-A 2 116 974 (3) bekannt. Die Oxidation gesättigter aliphatischer Kohlenwasserstoffe mit Wasserstoffperoxid an Titansilikaliten wird in der EP-A 376 453 (4) beschrieben.

Die Syntheseroute über die Ausgangsstoffe Propen, Wasserstoff und Sauerstoff für die Herstellung von Propylenoxid beschreiben die DE-A 44 25 672 (5) sowie die in (5) zitierten Publikationen. Als Katalysatoren werden funktionalisierte Zeolithe eingesetzt.

In den genannten Schriften (1) bis (5) werden Netzgewebe als Träger nicht erwähnt. Netzkatalysatoren ohne Zeolithe sind jedoch aus anderen Anwendungen als der vorliegenden bekannt. Aus der EP-A 511 739 (6) sind weiterhin Membranen zur Anwendung in Trennverfahren bekannt, welche Aluminiumoxid-Träger mit darauf aufgezogenen Filmen aus Zeolithkristallkörpern darstellen. Yamazaki und Tsutsumi beschreiben in Microp. Mater. 5 (1995), S. 245-253 (7) die Herstellung und die Eigenschaften von Membranen aus Mordenit (einer Zeolith-Struktur) auf Edelstahl oder Polytetrafluorethylen. Zeolithe auf Kupferdrahtgeflechten als Reduktionskatalysatoren für die Abgasreinigung (DeNox) sind von Calis et al. aus Chem.-Ing.-Tech. 67 (1995), S. 777-780 (8) bekannt. Die in (6) bis (8) genannten Zeolithe sind jedoch frei von Titan oder Vanadium.

Solche Arten der Trägerung, wie sie in (6) bis (8) beschrieben sind, sind nun speziell für die Problemstellung eines Epoxidierungsverfahrens schon alleine deshalb nicht anwendbar, da Alkalimetall bereits in Spuren die katalytische Aktivität von Titanzeolithen bei Oxidationsreaktionen zunichte machen können. Beispielsweise geht man in (7) und (8) von alkalimetallhaltigen Zeolith-Synthesegelen aus.

Der vorliegenden Erfindung lag die Aufgabe zugrunde, verbesserte Katalysatoren, insbesondere für die Herstellung von Epoxiden aus Olefinen, bereitzustellen, welche die Nachteile des Standes der Technik nicht mehr aufweisen. Insbesondere sollen die Katalysatoren mit möglichst wenig Aufwand herstellbar sein und eine hohe Effizienz zeigen.

Demgemäß wurde ein Netzkatalysator auf Basis von Titanzeolithen vom Typ MFI, MEL, BEA, MTW, TON, MOR oder mit MFI/MEL-Mischstruktur und inerten Netzgeweben gefunden.

Als inerte Netzgewebe, d.h. Trägernetze, insbesondere Drahtgeflechte, kommen vorzugsweise solche aus Metallen wie Edelstahl, Kupfer, Silber, Aluminium oder Nickel oder Legierungen wie Messing oder Kanthal (Eisen/Chrom/Aluminium/Kobalt), Kunststoffen wie Polytetrafluorethylen, Aluminiumoxiden wie α-Aluminiumoxid, Glasfasern, Kohlefasern oder Graphit in Betracht. Es können auch mehrere der genannten Netzgewebematerialien nebeneinander verwendet werden.

Da bereits Spuren von Alkalimetallen die katalytische Aktivität der Titanzeolithe bei Oxidationsreaktionen zunichte machen können, weist der erfindungsgemäße Netzkatalysator vorzugsweise einen Alkalimetallgehalt (insbesondere sind hier Natrium und/oder Kalium gemeint) von weniger als 500 ppm, insbesondere von weniger als 100 ppm, vor allem von weniger als 40 ppm auf, jeweils bezogen auf die Gewichtsmenge der Titanzeolithe. Derart niedrige Alkalimetallgehalte können durch entsprechende Herstellmethoden erzielt werden.

Zeolithe sind bekanntermaßen kristalline Alumosilikate mit geordneten Kanal- und Käfigstrukturen, deren Porenöffnungen im Bereich von Mikroporen, die kleiner als 0,9 nm sind, liegen. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson, "Atlas of Zeolite Structure Types", Butterworth, 2nd Ed., London 1987.

Es sind nun auch Zeolithe bekannt, die kein Aluminium enthalten und bei denen im Silikatgitter anstelle des Si(IV) teilweise Titan als Ti(IV) steht. Diese Titanzeolithe, insbesondere solche mit einer Kristallstruktur vom MFI-Typ, sowie Möglichkeiten zu ihrer Herstellung sind beschrieben, beispielsweise in der EP-A 311 983 oder der EP-A 405 978. Außer Silizium und Titan können solche Materialien auch zusätzliche Elemente wie Aluminium, Gallium, Bor oder geringe Mengen an Fluor enthalten.

Im erfindungsgemäßen Netzkatalysator kann das Titan des Zeoliths teilweise durch Vanadium ersetzt sein. Das molare Verhältnis von Titan und/oder Vanadium zur Summe aus Silicium plus Titan und/ oder Vanadium liegt in der Regel im Bereich von 0,01:1 bis 0,1:1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich bei etwa 960 cm⁻¹ identifiziert werden können und sich damit von Alkalimetalltitanaten oder kristallinen und amorphen TiO₂-Phasen unterscheiden.

Typischerweise stellt man die genannten Titanzeolithe dadurch her, daß man eine wäßrige Mischung aus einer SiO₂-Quelle, einer Titan-Quelle wie Titandioxid und einer stickstoffhaltigen organischen Base ("Schablonen-Verbindung"), z.B. Tetrapropylammoniumhydroxid, in einem Druckbehälter unter erhöhter Temperatur im Zeitraum mehrerer Stunden oder einiger Tage umsetzt, wobei das kristalline Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan zumindest teilweise innerhalb des Zeolithgerüsts in wechselnden Anteilen mit vier-, fünf- oder sechsfacher Koordination vor. Zur Verbesserung des katalytischen Verhaltens kann sich noch eine mehrmalige Waschbehandlung mit schwefelsaurer Wasserstoffperoxidlösung anschließen, worauf das Titanzeolith-Pulver erneut getrocknet und gebrannt werden muß. Der pulverförmige Titanzeolith müßte nun gemäß dem Stand der Technik abschließend in Zusätzen mit einem geeigneten inerten Binder in einem Formgebungsschritt verarbeitet werden, um ihn als Katalysator in einer handhabbaren Form verfügbar zu machen.

Bei der Verwendung von Titanzeolithen vom Typ MFI, BEA, MTW, TON oder MOR als Katalysatoren ergeben sich aber hierbei einige schwerwiegende Nachteile.

Die Verarbeitung der hergestellten Titanzeolith-Pulver in Formgebungsschritten zur Fertigung konventionell handhabbarer Katalysatoren ist arbeitsaufwendig und erfordert nach einer Verstrangung mit oder ohne einen inerten Binder einen weiteren energieintensiven und zeitaufwendigen Trocknungs- und Kalzinierschritt. Auch das Aufbringen großer Zeolithkristalle über einen Schmelz- oder Sintervorgang ist nachteilig. Darüberhinaus ergeben sich bei einer Festbettanordnung der konventionell verformten Titanzeolithe Probleme beim Erzielen eines hohen Katalysatornutzungsgrades einerseits und der Wunsch nach geringerem Druckverlust längs des Reaktors andererseits. Bei Oxidationen in der Gasphase sind zudem hohe Gasgeschwindigkeiten wünschenswert, um durch kurze Verweilzeiten Folgereaktionen vermeiden zu können. - Diese Probleme sind durch die vorliegende Erfindung gelöst worden.

Bevorzugte Titanzeolithe sind die in (5) beschriebenen Titansilikalite mit Zeolith-Struktur, vorzugsweise mit Pentasil-Zeolith-Struktur, insbesondere die Typen mit röntgenographischer Zuordnung zur MFI-, MEL- oder MFI/MEL-Mischstruktur. Zeolithe dieses Typs sind beispielsweise in W.M. Meier und D.H. Olson, "Atlas of Zeolite Structure Types", Butterworths, 2nd Ed., London 1987, beschrieben. Denkbar sind für die vorliegende Erfindung weiterhin titanhaltige Zeolithe mit der Struktur des ZSM-48, ZSM-12, Ferrierit oder β-Zeolith und des Mordenits.

In einer weiteren bevorzugten Ausführungsform enthält der erfindungsgemäße Netzkatalysator zusätzlich 0,01 bis 30 Gew.-%, insbesondere 0,05 bis 15 Gew.-%, vor allem 0,1 bis 8 Gew.-%, jeweils bezogen auf die Menge der Titanzeolithe, an einem oder mehreren Edelmetallen aus der Gruppe Gold, Silber, Rhenium, Ruthenium, Rhodium, Palladium, Osmium, Iridium und Platin. Besonders bevorzugt ist die Mitverwendung der genannten Edelmetalle in den genannten Mengen beim Einsatz von Titansilikaliten mit Zeolith-Struktur.

Im Sinne der vorliegenden Erfindung werden Titanzeolithe, gegebenenfalls zusammen mit Edelmetallen oder Edelmetalle enthaltenden Verbindungen, auf inertes Netzgewebe, vorzugsweise durch Kontaktieren in einer hydrothermalen Umsetzung einer SiO₂-Quelle mit einer Titankomponente in Gegenwart wäßriger Lösungen von schwach konzentrierten Tetraalkylammoniumhalogeniden und zusätzlich Ammoniak oder von Tetraalkylammoniumhydroxiden, aufkristallisiert. Dabei ermöglicht es das erfindungsgemäße Verfahren, daß man die entstehenden Titanzeolith-Kristalle in hoher Ausbeute direkt in Form plättchenförmiger, alkalimetallfreier Primärkristallite erhält. Nach dem erfindungsgemäßen Verfahren erhält man katalytisch verwendbare Titansilikat-Zeolithe des Strukturtyps MFI, MEL, MTW, BEA, TON oder MOR vorzugsweise dadurch, daß man die Titankomponente in Form einer löslichen, wäßrigen oder wäßrig-alkoholischen Titanverbindung in der vorbeschriebenen Weise bei der hydrothermalen Umsetzung der Reaktionsmischung zusetzt.

Das erfindungsgemäße alkalimetallfreie Herstellverfahren für die Netzkatalysatoren macht es möglich, daß darüber hinaus das Material nach einer Temperaturbehandlung bei 350 bis 600°C, vorzugsweise bei 400 bis 550°C und insbesondere bei 450 bis 500°C direkt und ohne zusätzlichen Ionenaustausch auf den Netzgeweben in einer katalytisch wirksamen Form vorliegt und als Katalysator verwendet werden kann.

Der Gehalt der erfindungsgemäßen Netzkatalysatoren an katalytisch aktivem Material, d. h. an gegebenenfalls edelmetallhaltigen Titanzeolithen, beträgt üblicherweise 1 bis 30 Gew.-%, insbesondere 5 bis 20 Gew.-%.

Die titanzeolithbeschichteten Netzgewebe können nun ihrerseits nach an sich bekannten Methoden zu Monolithen verformt werden, die sich direkt der Reaktorgeometrie optimal anpassen.

Ebenso können die erfindungsgemäßen Netzkatalysatoren vorteilhaft dann verwendet werden, wenn Reaktionswärmen über den gut leitenden, beispielsweise metallischen Körper der Netzkatalysatoren schnell zu- oder abgeführt werden sollen oder wenn die monolithische Netzpackung in einem durchströmten Reaktor zusätzlich die Funktion eines statischen Mischers übernehmen oder bei hohen Gasgeschwindigkeiten in der Gasphase einen möglichst geringen Druckverlust längs des Reaktors gewährleisten soll.

Die Herstellung der vorliegenden erfindungsgemäßen Netzkatalysatoren ist deshalb besonders einfach, weil die Überführung der inerten Träger in die erfindungsgemäßen Netzkatalysatoren üblicherweise bereits im Verfahrensschritt der Kristallisation der Titanzeolithe erfolgt.

Die erfindungsgemäßen Netzkatalysatoren auf Basis von Titanzeolithen und inerten Netzgeweben eignen sich in hervorragender Weise zur Beschleunigung von Oxidationsreaktionen. Unter Oxidationsreaktionen sind dabei insbesondere Epoxidierungen von olefinischen Doppelbindungen, die Oxidation von Wasserstoff zu Wasserstoffperoxid, Ammoxidationsreaktionen und Hydroxylierungen, beispielsweise von Aromaten zu Phenolen, zu verstehen.

Demgemäß ist auch Gegenstand der vorliegenden Erfindung ein Verfahren zur Herstellung von Epoxiden aus Olefinen mittels Sauerstoff oder sauerstoffliefernder Verbindungen, welches dadurch gekennzeichnet ist, daß man die Olefine in Gegenwart der erfindungsgemäßen Netzkatalysatoren auf Basis von Titanzeolithen und inerten Netzgeweben umsetzt.

Abhängig vom umzusetzenden Olefin und von der Sauerstoff-Quelle kann die erfindungsgemäße Epoxidierung in flüssiger Phase, in der Gasphase oder auch in überkritischer Phase durchgeführt werden. Wird die Epoxidierung beispielsweise mit wäßrigem Wasserstoffperoxid durchgeführt, arbeitet man zweckmäßigerweise in flüssiger Phase, wogegen eine Umsetzung der Olefine beispielsweise mit einem Wasserstoff/Sauerstoff-Gasgemisch in einer Flüssigphasen- oder einer Gasphasen-Fahrweise erfolgen kann.

Wird die Epoxidierung beispielsweise mit wäßrigem Wasserstoffperoxid, z.B. 10 bis 50 gew.-%igem wäßrigen Wasserstoffperoxid, als sauerstoffliefernder Verbindung durchgeführt, führen schon die erfindungsgemäßen Netzkatalysatoren auf Basis von Titanzeolithen allein ohne Edelmetallgehalt zu hervorragenden Ergebnissen. Arbeitet man dagegen beispielsweise mit einem Wasserstoff/Sauerstoff-Gasgemisch als Sauerstoff-Quelle, empfiehlt sich in vielen Fällen die Mitverwendung der genannten Edelmetalle in den erfindungsgemäßen Netzkatalysatoren; insbesondere werden hierbei edelmetallhaltige Netzkatalysatoren bevorzugt, welche Titansilikalite mit Zeolith-Struktur enthalten.

Wird die erfindungsgemäße Epoxidierung in flüssiger Phase vorgenommen, arbeitet man vorteilhafterweise bei einem Druck von 1 bis 10 bar und in Gegenwart von Lösungsmitteln. Als Lösungsmittel eignen sich Alkohole, z.B. Methanol, Ethanol, iso-Propanol oder tert.-Butanol oder Mischungen hieraus und insbesondere Wasser. Man kann auch Mischungen der genannten Alkohole mit Wasser einsetzen. In bestimmten Fällen bewirkt die Verwendung von Wasser oder wasserhaltigen Lösungsmittelsystemen eine deutliche Selektivitätssteigerung des gewünschten Epoxids gegenüber den reinen Alkoholen als Lösungsmittel.

Die erfindungsgemäße Epoxidierung wird in der Regel bei Temperaturen von -20 bis 70°C, insbesondere -5 bis 50°C, vorgenommen. Bei Verwendung eines Wasserstoff/Sauerstoff-Gasgemisches kann das Molverhältnis von Wasserstoff zu Sauerstoff üblicherweise im Bereich H₂:O₂ = 1:10 bis 1:1 variiert werden und ist besonders günstig bei 1:5 bis 1:1. Das molare Verhältnis von Sauerstoff zu Olefin liegt hierbei in der Regel bei 1:4 bis 1:10, vorzugsweise 1:5 bis 1:7. Als Trägergas kann ein beliebiges Inertgas zugefahren werden, insbesondere eignet sich Stickstoff.

Das eingesetzte Olefin kann eine beliebige organische Verbindung sein, die mindestens eine ethylenisch ungesättigte Doppelbindung enthält. Sie kann aliphatischer, aromatischer oder cycloaliphatischer Natur sein, sie kann aus einer linearen oder einer verzweigten Struktur bestehen. Vorzugsweise enthält das Olefin 2 bis 30 C-Atome. Mehr als eine ethylenisch ungesättigte Doppelbindung kann vorhanden sein, so etwa in Dienen oder Trienen. Das Olefin kann zusätzlich funktionelle Gruppe wie Halogenatome, Carboxylgruppen, Carbonesterfunktionen, Hydroxylgruppen, Etherbrücken, Sulfidbrücken, Carbonylfunktionen, Cyanogruppen, Nitrogruppen oder Aminogruppen enthalten.

Typische Beispiele für derartige Olefine sind Ethylen, Propen, 1-Buten, cis- und trans-2-Buten, 1,3-Butadien, Pentene, Isopren, Hexene, Octene, Nonene, Decene, Undecene, Dodecene, Cyclopenten, Cyclohexen, Dicyclopentadien, Methylencyclopropan, Vinylcyclohexan, Vinylcyclohexen, Allylchlorid, Acrylsäure, Methacrylsäure, Crotonsäure, Vinylessigsäure, Allylalkohol, Alkylacrylate, Alkylmethacrylate, Ölsäure, Linolsäure, Linolensäure, Ester und Glyceride derartiger ungesättigter Fettsäuren, Styrol, α-Methylstyrol, Divinylbenzol, Inden und Stilben. Auch Mischungen der genannten Olefine können nach dem erfindungsgemäßen Verfahren epoxidiert werden.

Das erfindungsgemäße Verfahren eignet sich in besonderem Maße für die Epoxidierung von Propen zu Propylenoxid.

Gegenstand der vorliegenden Erfindung ist auch ein Verfahren zur Herstellung von Wasserstoffperoxid aus Wasserstoff und Sauerstoff, welches dadurch gekennzeichnet ist, daß man die Umsetzung
- in der Regel heterogenkatalytisch - unter Verwendung der erfindungsgemäßen Netzkatalysatoren auf Basis von Titanzeolithen und inerten Netzgeweben vornimmt. Dabei erzielt man insbesondere mit Netzkatalysatoren, welche Titansilikalite mit Zeolith-Struktur und vorzugsweise zusätzlich 0,01 bis 30 Gew.-%, bezogen auf die Menge der Titansilikalite, eines oder mehrerer der oben genanten Edelmetalle enthalten, hervorragende Ergebnisse.

Gegenstand der vorliegenden Erfindung ist weiterhin ein Verfahren zur Herstellung von Hydroxylaminen aus Ammoniak oder den entsprechenden Aminen, Wasserstoff und Sauerstoff, welches dadurch gekennzeichnet ist, daß man die Umsetzung in Gegenwart von Netzkatalysatoren auf Basis von Titanzeolithen und inerten Netzgeweben vornimmt. Dabei erzielt man insbesondere mit Netzkatalysatoren, welche Titansilikalite mit Zeolith-Struktur und vorzugsweise zusätzlich 0,01 bis 30 Gew.-%, bezogen auf die Menge der Titansilikalite, eines oder mehrerer der oben genanten Edelmetalle enthalten, hervorragende Ergebnisse.

Der erfindungsgemäße Netzkatalysator eignet sich insbesondere zur Herstellung von unsubstituiertem Hydroxylamin, daneben aber auch zur Herstellung von substituierten Hydroxylaminen aus den entsprechenden Aminen, Wasserstoff und Sauerstoff, z.B. aus cyclischen oder aliphatischen Aminen wie Cyclohexylamin, welche unter den Reaktionsbedingungen teilweise zu den entsprechenden Lactamen weiterreagieren können.

Werden Hydroxylamine in flüssiger Phase hergestellt, arbeitet man vorteilhafterweise bei einem Druck von 1 bis 100 bar und in Gegenwart von Lösungsmitteln. Als Lösungsmittel eignen sich Alkohole, z.B. Methanol, Ethanol, iso-Propanol oder tert.-Butanol oder Mischungen hieraus und insbesondere Wasser. Man kann auch Mischungen der genannten Alkohole mit Wasser einsetzen. In bestimmten Fällen bewirkt die Verwendung von Wasser oder wasserhaltigen Lösungsmittelsystemen eine deutliche Selektivitätssteigerung des gewünschten Produktes gegenüber den reinen Alkoholen als Lösungsmittel.

Die erfindungsgemäße Umsetzung zu den gewünschten Hydroxylaminen wird in der Regel bei Temperaturen von -5 bis 70°C, insbesondere 20 bis 50°C, vorgenommen. Das Molverhältnis von Wasserstoff zu Sauerstoff kann üblicherweise im Bereich H₂:O₂ = 1:10 bis 1:1 variiert werden und ist besonders günstig bei 1:2,5 bis 1:1. Das molare Verhältnis von Sauerstoff zum Ammoniak liegt in der Regel bei 1:1 bis 1:3, vorzugsweise 1:1,5 bis 1:1,7. Als Trägergas kann ein beliebiges Inertgas zugefahren werden, insbesondere eignet sich Stickstoff.

Die nachfolgenden Beispiele sollen das erfindungsgemäße Herstellverfahren für die beschriebenen Netzkatalysatoren sowie die katalytischen Eigenschaften der erfindungsgemäßen Netzkatalysatoren erläutern, ohne daß dadurch jedoch eine Beschränkung zu verstehen wäre.

### Beispiel 1

Das Beispiel beschreibt die Herstellung eines erfindungsgemäßen Netzkatalysators mit Edelstahlnetzen als Trägermaterial.

In einem Rührkolben wurden 329 g deionisiertes Wasser vorgelegt und unter Rühren 22,7 g Tetraisopropylorthotitanat innerhalb einer halben Stunde zugetropft. Die weiße Suspension wurde auf 5°C abgekühlt. Dazu gab man 272 g Wasserstoffperoxidlösung (30 Gew.-% in Wasser) und rührte die orangerote Lösung noch 39 Minuten nach. Man fügte 704 g Ammoniaklösung (25 Gew.-% in Wasser) zu und hielt die gelbe Suspension für 15 Stunden bei Raumtemperatur. Abschließend erhitzte man 3 Stunden auf 80°C, um überschüssiges Wasserstoffperoxid zu zersetzen.

In einem Polypropylenbecher homogenisierte man 58,8 g Tetrapropylammoniumbromid, 88,7 g Silica (Aerosil® 200 der Fa. Degussa), 122,4 g deionisiertes Wasser und 626 g der vorgenannten Titanat-Hydrolysatsuspension.

In einen Autoklaven-Becher mit Teflon-Auskleidung gab man zwei Netzstücke von 10,8 g bzw. 11,9 g eines Edelstahl-Drahtgewebenetzes (Stahl 1.4401) und füllte mit 150 g des obigen Titanzeolith-Syntheseansatzes auf. Bei 175°C wurde innerhalb eines Zeitraumes von 168 Stunden kristallisiert.

Nach Beendigung der Kristallisation wurden die Netze entfernt, mit Wasser neutralgewaschen, bei 120°C getrocknet und bei 500°C unter synthetischer Luft (3 Vol-% Sauerstoff-Anteil, 97 Vol-% Stickstoff) kalziniert. Lose anhaftende Gelreste wurden mittels Druckluftstrahl entfernt. Die Gewichtszunahme durch aufkristallisierten Titanzeolith betrug 8,7 bzw. 8,0 Gew.-% bei den beiden Teilstücken. Diese Netze wurden ohne weitere Vorbehandlung in der Propylenoxid-Erzeugung von Beispiel 3 eingesetzt.

### Vergleichsbeispiel A

Dieses Vergleichsbeispiel beschreibt die Herstellung eines Titansilikalits mit MFI-Struktur, wie es nach dem Stand der Technik in EP 376 453 (4) beschrieben ist.

30 ml Tetraethylorthotitanat wurden unter Rühren (300 U/min) innerhalb von 15 Minuten in 375 ml deionisiertes Wasser getropft, welches zuvor auf 2°C abgekühlt worden war. Danach wurde mit 360 ml kalter Wasserstoffperoxidlösung (30 Gew.-% in Wasser) versetzt, worauf sich eine orangerote Lösung bildete, die für die Dauer von 2 Stunden gerührt wurde. Danach wurden 625 ml wäßrige Tetrapropylammoniumhydroxidlösung (20 Gew.-% in Wasser) und nach einer weiteren Stunden 100 g kolloidale Silicasol-Lösung (40 Gew.-% SiO₂, Ludox® AS-40 der Fa. Du Pont) zugegeben. Diese Mischung wurde über Nacht bei Raumtemperatur aufbewahrt, am nächsten Tag unter Rühren (300 U/min) für die Dauer von 7 Stunden auf 80°C erhitzt, in einen 2 l fassenden Druckrührbehälter eingefüllt und für die Dauer von 240 Stunden bei 175°C zur Reaktion gebracht.

Das erkaltete Reaktionsgemisch wurde abfiltriert, der Filterkuchen mehrmals mit deionisiertem Wasser neutralgewaschen, bei 120°C über Nacht getrocknet und abschließend bei 550°C in Luftatmosphäre kalziniert. Bezogen auf eingesetztes SiO₂ betrug die Ausbeute an Titansilikalit 93 %. Nach dem Röntgenbeugungsmuster handelte es sich um reinen Titansilikalit mit MFI-Struktur.

### Beispiel 2

Dieses Beispiel beschreibt die Herstellung eines erfindungsgemäßen Netzkatalysators it Graphitnetzen als Trägermaterial.

In einem Rührkolben wurden 329 g deionisiertes Wasser vorgelegt und unter Rühren 22,7 g Tetraisopropylorthotitanat innerhalb einer halben Stunde zugetropft. Die weiße Suspension wurde auf 5°C abgekühlt. Dazu gab man 272 g Wasserstoffperoxidlösung (30 Gew.-% in Wasser) und rührte die orangerote Lösung noch 30 Minuten nach. Man fügte 704 g Ammoniaklösung (25 Gew.-% in Wasser) zu und hielt die gelbe Suspension für 15 Stunden bei Raumtemperatur. Abschließend erhitzte man 3 Stunden auf 80°C, um überschüssiges Wasserstoffperoxid zu zersetzen.

In einem Polypropylenbecher homogenisiert man 58,8 g Tetrapropylammoniumbromid, 88,7 g Silica (Aerosil® 200 der Fa. Degussa), 122,4 g deionisiertes Wasser und 626 g der vorgenannten Titanat-Hydrolysatsuspension.

In einen Autoklaven-Becher mit Teflon-Auskleidung gab man zwei Netzstücke von 5,2 g bzw. 5,3 g eines Graphitgewebenetzes (Fa. KDL 8042, 200 g/m²) und füllte mit 150 g des obigen Titanzeolith-Syntheseansatzes auf. Bei 175°C wurde innerhalb eines Zeitraumes von 168 Stunden kristallisiert.

Nach Beendigung der Kristallisation wurden die Netze entfernt, mit Wasser neutralgewaschen, bei 120°C getrocknet und bei 500°C unter Stickstoff kalziniert. Lose anhaftende Gelreste werden mittels Druckluftstrahl entfernt. Die Gewichtszunahme durch aufkristallisierten Titanzeolith betrug 10,4 bzw. 8,7 Gew.-% bei den beiden Teilstücken. Diese Netze wurden ohne weitere Vorbehandlung in der Propylenoxid-Erzeugung von Beispiel 4 eingesetzt.

### Vergleichsbeispiele B und C

Diese Synthesebeispiele beschreiben den Einfluß beim Wechsel von Tetrapropylammoniumhydroxid zu Tetrapropylammoniumbromid unter gleichzeitiger Verwendung von Ammoniaklösung.

In einem mit Rührer und Rückflußkühler versehenen Glaskolben wurden 45,1 g deionisiertes Wasser auf 5°C abgekühlt. Dazu tropfte man innerhalb von 15 Minuten 6,9 g Tetraisopropylorthotitanat und 81,4 g Wasserstoffperoxidlösung (30 Gew.-% in Wasser). Zu der entstandenen orangeroten Lösung fügte man 211,0 g einer Ammoniaklösung (25 Gew.-% in Wasser) und ließ den entstandenen Ansatz sich über Nacht auf Raumtemperatur erwärmen. Abschließend wurde für die Dauer von 3 Stunden auf 80°C unter Rühren erwärmt. Eventuell eintretender Gewichtsverlust wurde durch Zugabe einer entsprechenden Menge an Ammoniaklösung ausgeglichen.

Von dieser so vorbereiteten Lösung wurden 156,8 g mit 56,0 g Tetrapropylammoniumhydroxidlösung (20 Gew.-% in Wasser) und 52,8 g Ludox® AS-40 Silicasol (Du Pont) innerhalb 3 Minuten gemischt, in einen teflonausgekleideten Autoklavenbehälter eingefüllt und druckdicht verschlossen (im folgenden als Ansatz B bezeichnet).

Weitere 156,5 g der eingangs vorbereiteten Lösung wurden mit 14,7 g Tetrapropylammoniumbromid in 44,8 g Wasser und 53,1 g Ludox® AS-40 Silicasol (Du Pont) innerhalb von 3 Minuten gemischt, in einen teflonausgekleideten Autoklavenbehälter eingefüllt und druckdicht verschlossen (im folgenden als Ansatz C bezeichnet).

Die Ansätze B und C wurden jeweils bei einer Temperatur von 183 bis 185°C innerhalb von 192 Stunden zur Reaktion gebracht. Die kristallinen Reaktionsprodukte wurden abfiltriert, neutralgewaschen, getrocknet und an Luft bei 500°C für die Dauer von 5 Stunden kalziniert. Die Eigenschaften beider Vergleichsbeispiele sind in der nachfolgenden Tabelle gegenübergestellt.

| Ansatz | Ausbeute | Si/Ti [molar] | Kalium [Gew.-%] | Größe [µm] | Form |
|---|---|---|---|---|---|
| B | 96 % | 36 | 0,001 | 0,1 | globulär |
| C | 95 % | 36 | 0,001 | 26 | plättchenförmig |

### Beispiel 3

Dieses Beispiel beschreibt die erfindungsgemäße Propylenoxid-Erzeugung mit dem Katalysator aus Eeispiel 1.

In einem Glasdruckautoklaven wurden 9 g Katalysator-Netz aus Beispiel 1 in 45 ml Methanol suspendiert. Nach dem Abkühlen des Autoklaven auf -30°C wurden 20,5 g Propen aufgepreßt, danach brachte man den Autoklaven auf 0°C. Nun gab man 30 g einer 30 gew.-%igen wäßrigen H₂O₂-Lösung innerhalb von 20 min zu und rührte die Reaktionsmischung weitere 5 h bei 0°C. Nach Ende der Reaktion wurde die gelöste Menge an Propylenoxid gaschromatographisch bestimmt. Es wurden 1,65 Gew.-% Propylenoxid gefunden.

### Beispiel 4

Dieses Beispiel beschreibt die erfindungsgemäße Propylenoxid-Erzeugung mit dem Katalysator aus Beispiel 2.

In einem Glasdruckautoklaven wurden 15 g Katalysator-Netz aus Beispiel 2 in 45 ml Methanol suspendiert. Nach dem Abkühlen der Autoklaven auf -30°C wurden 19,7 g Propen aufgepreßt, danach brachte man den Autoklaven auf 0°C. Nun gab man 40,6 g einer 30 gew.-%igen wäßrigen H₂O₂-Lösung innerhalb von 25 min zu und rührte die Reaktionsmischung weitere 5 h bei 0°C. Nach dem Ende der Reaktion wurde die Menge an gelöstem Propylenoxid gaschromatographisch mit 4,9 Gew.-% bestimmt.

Die Beispiele 1 bis 4 zeigen, daß die erfindungsgemäßen Netzkatalysatoren weniger aufwendig herzustellen sind und bessere Ergebnisse in der Epoxidierung liefern als mehrstufig konventionell geträgerte Titanzeolithe wie in Vergleichsbeispielen B bis E (s.u.) beschrieben.

### Vergleichsbeispiel D

Dieses Beispiel beschreibt die Propylenoxid-Erzeugung mit ungeformtem Katalysator aus Vergleichsbeispiel B.

In einem Glasdruckautoklaven wurden 1,5 g Katalysator aus Vergleichsbeispiel B in 45 ml Methanol suspendiert. Nach dem Abkühlen der Autoklaven auf -30°C werden 23,5 g Propen aufgepreßt, danach brachte man den Autoklaven auf 0°C. Nun gab man 28,9 g einer 30 gew.-%igen wäßrigen H₂O₂-Lösung innerhalb von 20 min zu und rührte die Reaktionsmischung weitere 5 h bei 0°C. Nach dem Ende der Reaktion wurde die Menge an gelöstem Propylenoxid gaschromatographisch mit 3,7 Gew.-% bestimmt.

### Vergleichsbeispiel E

Dieses Beispiel beschreibt die Propylenoxid-Erzeugung mit geformtem Katalysator aus Vergleichsbeispiel B.

100 g Katalysator aus Vergleichsbeispiel B wurden mit 12,5 g Ludox® AS-40, 5 g Methylcellulose und 70 ml Wasser 120 min lang im Kneter verdichtet und zu Strängen mit 2 mm Durchmesser verarbeitet. Die Stränge wurden bei 110°C über Nacht getrocknet und bei 500°C 5 h lang kalziniert. Die so erhaltenen Stränge wurden zu Splitt (Teilchenengröße ca. 1 mm) verarbeitet.

In einem Glasdruckautoklaven wurden 1,5 g dieses Katalysatorsplitts in 45 ml Methanol suspendiert. Nach dem Abkühlen des Autoklaven auf -30°C wurden 29,7 g Propen aufgepreßt, danach brachte man den Autoklaven auf 0°C. Innerhalb von 20 min wurden nun 31 g einer 30 gew.-%igen wäßrigen H₂O₂-Lösung zugegeben und die Reaktionsmischung wurde weiter 5 h gerührt. Nach Ende der Reaktion wurde die Menge an gelöstem Propylenoxid gaschromatographisch bestimmt. Es wurden 2,6 Gew.-% an Propylenoxid gefunden.

Dies belegt, daß beim Verformen ein wesentlicher Anteil der katalytischen Aktivität (siehe Vergleichsbeispiel D mit 3,7 Gew.-% Propylenoxid) des Ursprungsmaterials verlorengeht.

## Patentansprüche

1. Netzkatalysator auf Basis von Titanzeolithen vom Typ MFI, MEL, BEA, MTW, TON, MOR oder mit MFI/MEL-Mischstruktur und inerten Netzgeweben.

2. Netzkatalysator nach Anspruch 1 auf Basis von inerten Netzgeweben aus Metallen, Kunststoffen, Aluminiumoxiden, Glasfasern, Kohlefasern und/oder Graphit.

3. Netzkatalysator nach Anspruch 1 oder 2 mit einem Alkalimetallgehalt von weniger als 500 ppm, bezogen auf die Gewichtsmenge der Titanzeolithe.

4. Netzkatalysator nach den Ansprüchen 1 bis 3, enthaltend Titansilikalite mit Zeolith-Struktur und 0,01 bis 30 Gew.-%, bezogen auf die Menge der Titansilikalite, an einem oder mehreren Edelmetallen aus der Gruppe Gold, Silber, Rhenium, Ruthenium, Rhodium, Palladium, Osmium, Iridium und Platin.

5. Verfahren zur Herstellung eines Netzkatalysators gemäß den Ansprüchen 1 bis 4, dadurch gekennzeichnet, daß man Titanzeolithe und gegebenenfalls Edelmetalle oder Edelmetalle enthaltende Verbindungen auf inertes Netzgewebe aufkristallisiert.

6. Verwendung von Netzkatalysatoren auf Basis von Titanzeolithen vom Typ MFI, MEL, BEA, MTW, TON, MOR oder mit MFI/MEL-Mischstruktur und inerten Netzgeweben zur Beschleunigung von Oxidationsreaktionen.

7. Verfahren zur Herstellung von Epoxiden aus Olefinen mittels Sauerstoff oder sauerstoffliefernder Verbindungen, dadurch gekennzeichnet, daß man die Olefine in Gegenwart von Netzkatalysatoren auf Basis von Titanzeolithen vom Typ MFI, MEL, BEA, MTW, TON, MOR oder mit MFI/MEL-Mischstruktur und inerten Netzgeweben umsetzt.

8. Verfahren zur Herstellung von Propylenoxid nach Anspruch 7.

9. Verfahren zur Herstellung von Wasserstoffperoxid aus Wasserstoff und Sauerstoff, dadurch gekennzeichnet, daß man die Umsetzung in Gegenwart von Netzkatalysatoren auf Basis von Titanzeolithen vom Typ MFI, MEL, BEA, MTW, TON, MOR oder mit MFI/MEL-Mischstruktur und inerten Netzgeweben vornimmt.

10. Verfahren zur Herstellung von Hydroxylaminen aus Ammoniak oder den entsprechenden Aminen, Wasserstoff und Sauerstoff, dadurch gekennzeichnet, daß man die Umsetzung in Gegenwart von Netzkatalysatoren auf Basis von Titanzeolithen vom Typ MFI, MEL, BEA, MTW, TON, MOR oder mit MFI/MEL-Mischstruktur und inerten Netzgeweben vornimmt.

## Claims

1. A gauze catalyst based on titanium zeolites of the MFI, MEL, BEA, MTW, TON or MOR type or having an MFI/MEL mixed structure and inert gauze fabrics.

2. A gauze catalyst as claimed in claim 1 based on inert gauze fabrics made of metal, plastic, aluminum oxide, glass fiber, carbon fiber and/or graphite.

3. A gauze catalyst as claimed in claim 1 or 2 having an alkali metal content of less than 500 ppm, based on the weight of the titanium zeolites.

4. A gauze catalyst as claimed in any of claims 1 to 3 comprising titanium silicalites having a zeolite structure and from 0.01 to 30% by weight, based on the amount of the titanium silicalites, of one or more noble metals from the group consisting of gold, silver, rhenium, ruthenium, rhodium, palladium, osmium, iridium and platinum.

5. A process for preparing a gauze catalyst as claimed in any of claims 1 to 4, which comprises crystallizing titanium zeolites and, if desired, noble metals or noble metal-containing compounds onto an inert gauze fabric.

6. The use of gauze catalysts based on titanium zeolites of the MFI, MEL, BEA, MTW, TON or MOR type or having an MFI/MEL mixed structure and inert gauze fabrics for accelerating oxidation reactions.

7. A process for preparing epoxides from olefins using oxygen or oxygen-containing compounds, which comprises converting the olefins in the presence of a gauze catalyst based on titanium zeolites of the MFI, MEL, BEA, MTW, TON or MOR type or having an MFI/MEL mixed structure and inert gauze fabrics.

8. A process for preparing propylene oxide as claimed in claim 7.

9. A process for preparing hydrogen peroxide from hydrogen and oxygen, which comprises conducting the reaction in the presence of a gauze catalyst based on titanium zeolites of the MFI, MEL, BEA, MTW, TON or MOR type or having an MFI/MEL mixed structure and inert gauze fabrics.

10. A process for preparing hydroxylamines from ammonia or the corresponding amines, hydrogen and oxygen, which comprises conducting the reaction in the presence of a gauze catalyst based on titanium zeolites of the MFI, MEL, BEA, MTW, TON or MOR type or having an MFI/MEL mixed structure and inert gauze fabrics.

## Revendications

1. Catalyseur réticulé à base de zéolites de titane du type MFI, MEL, BEA, MTW, TON, MOR ou ayant une structure mixte MFI/MEL et de tissus réticulés inertes.

2. Catalyseur réticulé selon la revendication 1 à base de tissus réticulés inertes constitués de métaux, de matières plastiques, d'oxydes d'aluminium, de fibres de verre, de fibres de carbone, et/ou de graphite.

3. Catalyseur réticulé selon la revendication 1 ou 2 ayant une teneur en métal alcalin inférieure à 500 ppm, par rapport à la quantité pondérale des zéolites de titane.

4. Catalyseur réticulé selon les revendications 1 à 3, contenant des silicalites de titane ayant une structure de zéolite et 0,01 à 30% en poids de un ou plusieurs métaux nobles choisis dans le groupe formé par l'or, l'argent, le rhénium, le ruthénium, le rhodium, le palladium, l'osmium, l'iridium et le platine, par rapport à la quantité des silicalites de titane.

5. Procédé de préparation d'un catalyseur réticulé selon les revendications 1 à 4, caractérisé en ce que l'on dépose par cristallisation des zéolites de titane et éventuellement des métaux nobles ou des composés contenant des métaux nobles sur un tissu réticulé inerte.

6. Utilisation de catalyseurs réticulés à base de zéolites de titane de type MFI, MEL, BEA, MTW, TON, MOR ou ayant une structure mixte MFI/MEL et de tissus réticulés inertes en vue d'accélérer des réactions d'oxydation.

7. Procédé de préparation d'époxydes à partir d'oléfines au moyen d'oxygène ou de composés livrant de l'oxygène, caractérisé en ce que l'on met à réagir les oléfines en présence de catalyseurs réticulés à base de zéolites de titane de type MFI, MEL, BEA, MTW, TON, MOR ou ayant une structure mixte MFI/MEL et de tissus réticulés inertes.

8. Procédé de préparation de l'oxyde de propylène selon la revendication 7.

9. Procédé de préparation du peroxyde d'hydrogène à partir d'hydrogène et d'oxygène, caractérisé en ce que l'on procède à la réaction en présence de catalyseurs réticulés à base de zéolites de titane de type MFI, MEL, BEA, MTW, TON, MOR ou ayant une structure mixte MFI/MEL et de tissus réticulés inertes.

10. Procédé de préparation d'hydroxylamines à partir d'ammoniac ou des amines correspondantes, d'hydrogène et d'oxygène, caractérisé en ce que l'on procède à la réaction en présence de catalyseurs réticulés à base de zéolites de titane de type MFI, MEL, BEA, MTW, TON, MOR ou ayant une structure mixte MFI/MEL et de tissus réticulés inertes.
